(19) Europäisches Patentamt
European Patent Office
Office européen des brevets

(11) Publication number: **0 603 958 A1**

# (12) EUROPEAN PATENT APPLICATION

(21) Application number: **93203559.5**

(22) Date of filing: **17.12.93**

(51) Int. Cl.5: **G01N 33/53**, G01N 33/546, G01N 33/543

(30) Priority: **21.12.92 US 994009**

(43) Date of publication of application: **29.06.94 Bulletin 94/26**

(84) Designated Contracting States: **DE FR GB**

(71) Applicant: **EASTMAN KODAK COMPANY**
**343 State Street**
**Rochester New York 14650-2201(US)**

(72) Inventor: **Wu, Annie Liu, c/o Eastman Kodak Company**
**Patent Department,**
**343 State Street**
**Rochester, New York 14650-2201(US)**

(74) Representative: **Aufflick, James Neil et al**
**Kodak Limited**
**Patent Department**
**Headstone Drive**
**Harrow Middlesex HA1 4TY (GB)**

## (54) Improvement of the dynamic range in specific binding assays.

(57) A specific binding assay method particularly useful for measuring ligands having a wide concentration range has been discovered comprising contacting a liquid sample containing a ligand to be measured with a first receptor $R_1$ specific for the ligand, $R_1$ being immobilized on a water-insoluble substrate; a labeled, unbound second receptor $R_2$ specific for the ligand; and (iii) an unlabeled, unbound third receptor $R_3$ specific for the ligand, wherein $R_2$ and $R_3$ are present in a ratio of $R_2$:$R_3$ of from about 1:50 to about 1:1000 and $R_3$ is the same as $R_1$, $R_2$, or a mixture thereof, to obtain a water-insoluble complex of $R_1$, ligand, and $R_2$; (b) separating the water-insoluble complex from the liquid sample and unreacted $R_2$; and (c) measuring either the amount of $R_2$ associated with the water-insoluble complex or the amount of unreacted $R_2$ as an indication of the amount of the ligand in the sample. An analytical element has also been discovered comprising a porous spreading zone and in the porous spreading zone, or a different zone, a first receptor $R_1$ specific to the ligand, $R_1$ being immobilized on a water-insoluble particulate substrate; a labeled, unbound second receptor $R_2$ specific for the ligand; and an unlabeled, unbound third receptor $R_3$ specific for the ligand, wherein $R_2$ and $R_3$ are present in a ratio of $R_2$:$R_3$ of from about 1:50 to about 1:1000, and $R_3$ is the same as $R_1$, $R_2$, or a mixture thereof.

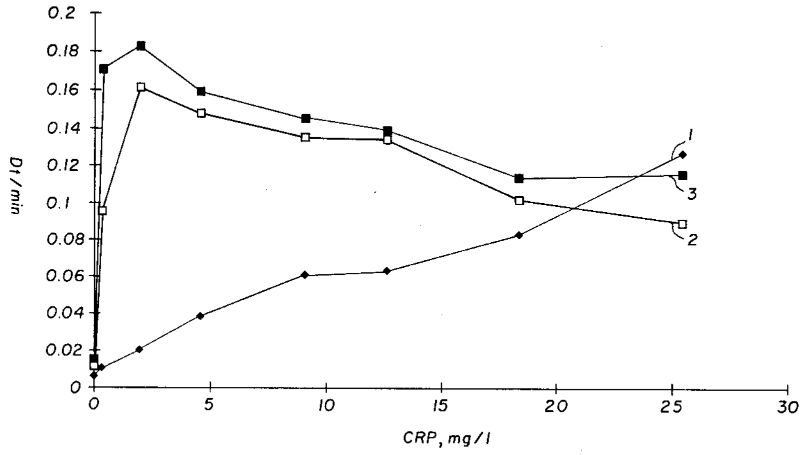


EP 0 603 958 A1

This invention relates to clinical chemistry. More specifically, this invention relates to a method for the improvement of the dynamic range in specific binding assays.

There are a number of analytes (termed "ligands" herein) that may be found over a variable range of concentration as present in biological specimens. The range of concentration in which the ligands are quantitatively measurable by the assay system is referred to herein as the dynamic range. While it is often a useful clinical tool, quantifying ligands having a wide concentration range is difficult.

Ligands having wide concentration ranges include, for example, acute phase proteins. The concentration of acute phase proteins changes dramatically upon a stimulus, such as, tissue injury, inflammation, and various infections. The concentration of the proteins typically parallels the course of injury or infection. Acute phase plasma proteins include, for example, ceruloplasmin, C3 (the third component of complement), $\alpha_1$-acid glycoprotein, $\alpha_1$-antitrypsin, $\alpha_1$-antichymotrypsin, fibrinogen, haptoglobin, C-Reactive protein (CRP), and serum amyloid A-protein (SAA). CRP and SAA have particularly wide concentration ranges. For example, CRP is normally a trace constituent in blood where the serum concentration in healthy adults normally remains below 5 mg/l. After the onset of a stimulus, the concentration rapidly and dramatically rises up to a 1000-fold or more. Upon healing or recovery of the patient and subsequent reduction of the stimulus, the concentration of CRP returns to the trace value. Monitoring disease activity by measuring the acute phase protein concentrations has become a much used practice in clinical chemistry.

Noncompetitive assays are useful tools in clinical chemistry. Problems have been encountered, however, in measuring ligands having a wide concentration range.

One type of noncompetitive assay typically involves the formation of a "sandwich" of the ligand between two specific binding receptors (and thus is commonly referred to as a sandwich assay). One of the receptors is attached to a water insoluble support and the other receptor is commonly labeled with a substance that generates a measurable signal. The two receptors typically recognize two spatially distinct sites on the ligand. Because one of the receptors is attached to the insoluble support, the entire "sandwich" of ligand and two receptors is also insoluble once formed. Consequently, the insoluble "sandwich" may be easily separated from soluble materials (including the unreacted labeled receptor). After separation, either the insoluble "sandwich" may be measured (or the unbound labeled receptor may be measured) to indicate the concentration of the ligand. The measurement of the signal system of the assay must be sensitive enough to quantify the targeted ligand at trace values as well as vastly higher concentrations without becoming saturated. Once saturation of the signal system occurs, meaningful quantitative analysis is limited. Typically to extend the dynamic range so that saturation will not occur requires dilution of the fluid sample and a second sandwich assay.

US-A-4,595,661 describes the use of additional entities in sandwich immunoassays when measuring antigens at high concentrations to avoid a "hook" effect, or false negative results at high concentrations. US-A-4,788,132 describes an adjustment in a sandwich immunoassay in order to obtain a pseudo first-order reaction which produces a linear standard curve.

Although many advances have been made in specific binding assays, it is highly desirable to discover an alternative specific binding sandwich assay system that is capable of quantifying ligands having a wide concentration range.

The present invention provides a specific binding assay method comprising (1) contacting a liquid sample containing a ligand to be measured with (a) a first receptor $R_1$ specific for the ligand, $R_1$ being immobilized on a water-insoluble substrate; (b) a labeled, unbound second receptor $R_2$ specific for the ligand; and (c) an unlabeled, unbound third receptor $R_3$ specific for the ligand, to obtain a water-insoluble complex of $R_1$, ligand, and $R_2$; (2) separating the water-insoluble complex from the liquid sample and unreacted $R_2$; and (3) measuring either the amount of $R_2$ associated with the water-insoluble complex or the amount of unreacted $R_2$ as an indication of the amount of the ligand in the sample,

characterized in that $R_2$ and $R_3$ are present in a ratio of $R_2$:$R_3$ of from 1:50 to 1:1000 and $R_3$ is the same as $R_1$, $R_2$, or a mixture thereof.

In a second embodiment, a novel analytical element has been discovered for measuring a ligand. The analytical element comprises a porous spreading zone and in the porous spreading zone, or a different zone, a first receptor $R_1$ specific to the ligand, $R_1$ being immobilized on a water-insoluble particulate substrate; a labeled, unbound second receptor $R_2$ specific for the ligand; and an unlabeled, unbound third receptor $R_3$ specific for the ligand,

characterized in that $R_2$ and $R_3$ are present in a ratio of $R_2$:$R_3$ of from 1:50 to 1:1000, and $R_3$ is the same as $R_1$, $R_2$, or a mixture thereof.

Unexpectedly, the ratio of $R_2$ to $R_3$ is instrumental in lowering the signal system generated in a specific binding assay. Consequently, this newly discovered method is particularly useful for measuring ligands having a wide concentration range. The targeted ligand may be quantified whether present in the specimen

at a very low or a very high concentration without requiring a dilution of the specimen. Thus, an improvement in the dynamic range of specific binding assays has been discovered.

The invention is illustrated by the accompanying drawing (Fig) which is a graph of transmission density (Dt/min) against C-reactive protein concentration (CRP,mg/l). The Figure the quantitative results achieved with the present invention (line 1) as compared with the saturation of the measurement signal systems resulting when employing prior art methods (lines 2 and 3), as described in Comparative Examples 1 and 2 below.

The present invention relates to the quantitative detection of a chemical or biological substance, termed a ligand herein. The term "ligand" is interchangeable with analyte, and includes, but is not limited to, antigens, haptens, antibodies, toxins, hormones, therapeutic drugs, natural and synthetic steroids, proteins, viruses, bacteria, peptides nucleotides, and so on.

The invention may be used to assay biological fluids or fluid preparations of human or animal tissue. Biological fluids that may be assayed include, but are not limited to, whole blood, plasma, sera, lymph, bile, urine, spinal fluid, sputum, perspiration and the like, as well as stool secretions. Fluid preparations of human or animal tissue that may be assayed include, for example, skeletal muscle, heart, kidney, lungs, brains, bone marrow, skin and so on.

More particularly, ligands measurable with the present invention include, but are not limited to, antigenic substances, such as,

1) serum proteins, such as,
alphafoetoprotein (AFP), cacinoembryonic antigen (CEA), immunoglobulin E (IgE), beta$_2$-microglobulin, thyroxine binding globulin (TBG), C-reactive protein (CRP), alpha$_2$-microglobulin, alpha$_1$-microglobulin, immunoglobulin A (IgA), immunoglobulin M (IgM), immunoglobulin G (IgG), immunoglobulin D (IgD), transferrine, sugar proteins, albumin, ceruloplasmin, C3 (the third component of complement), $\alpha_1$-acid glycoprotein, $\alpha_1$-antitrypsin, $\alpha_1$-antichymotrypsin, fibrinogen, haptoglobin, and serum amyloid A-protein (SAA), and the like,
2) hormones, such as, insulin, human chorionic gonadotrophin intact, alpha (intact or free), or beta-subunit (intact or free) (hCG, hCG-alpha, or hCG-beta) growth hormone, thyroid stimulating hormone (TSH), lutinizing hormone (LH), follicle stimulating hormone (FSH), prolactine, somatostatine, thyroxine, triiodothyronine, tetraiodothyronine, and the like,
3) tumor associated antigens, such as, ferritin, POA, CA-19-9, CA-12-5, and the like,
4) pathogens, including, for example, pathogenic bacteria (including mycobacteria, streptococcus, and so on), protozoas, parasites (including malarial parasite, toxoplasma gondii, and the like), viruses (including retroviruses, Epstein Barr virus, hepatitis viruses, cytomegalovirus, rubella virus, herpes virus, HIV-I and HTLV-I, and the like), and
5) enzymes, such as elastase, DNA-polymerase, amylase, proteinase, lipase, and the like.

The invention is particularly well-suited for ligands having a wide dynamic range, such as, for example, AFP, beta$_2$-microglobulin, TBG, IAP, CRP, alpha$_2$-microglobulin, IgA, IgM, IgG, IgD, IgE, ceruloplasmin, C3, $\alpha_1$-acid glycoprotein, $\alpha_1$-antitrypsin, $\alpha_1$-antichymotrypsin, fibrinogen, haptoglobin, SAA, and transferrine. Even more well-suited for the measurement by the inventive assay is CRP.

The receptors ($R_1$, $R_2$, and $R_3$) of the inventive assay may be any material that specifically binds to the targeted ligand, including, but not limited to, an antibody (including monoclonal, polyclonal, whole, fragments, derivatives, and mixtures thereof), antigen, avidin, biotin, thyroxin, thyroxin-binding globulin, polysaccharide, phosphorylcholine, aminoethyldihydrogen phosphate residues, estrogen, vitamin B-12 intrinsic factor, folate binding protein, sex hormone binding protein, mixtures thereof, and so on, each of which may be obtained from numerous known sources. The receptors may bind to the targeted ligand by an immuno reaction as well as a non-immuno reaction. As used herein, the receptors have been designated first receptor ("$R_1$"), second receptor ("$R_2$), and third receptor ("$R_3$"). The designations first, second, and third receptor have been used for clarification purposes rather than to indicate any kind of direction or mechanism of the assay system.

To serve as the capture agent, $R_1$ is immobilized on a water insoluble substrate. $R_2$ represents a labeled receptor. Although $R_1$ and $R_2$ may be selected from any materials that will specifically bind to the targeted ligand, $R_1$ and $R_2$ represent two different materials such that $R_1$ and $R_2$ specifically bind to two different sites on the targeted ligand. $R_3$ represents an unbound and unlabeled receptor that is defined as unbound $R_1$, a fragment of unbound $R_1$, unlabeled $R_2$, a fragment of unlabeled $R_2$, derivatives thereof, or a mixture thereof.

Many variations of targeted ligands and receptor types are included in this invention. For example, one preferred assay operates by immuno reactions such that the ligand targeted is an antigenic substance; $R_1$ is a polyclonal antibody specific to the antigenic substance; $R_2$ is a labeled monoclonal antibody specific to

the antigenic substance; and $R_3$ is the same monoclonal antibody as $R_2$ (with $R_3$ differing from $R_2$ only by virtue of being unlabeled). Alternatively, another preferred assay (especially suited for the measurement of CRP) combines both the immuno and non-immuno mechanisms such that the ligand is CRP, $R_1$ is phosphorylcholine, $R_2$ is a labeled monoclonal antibody, and $R_3$ is the same monoclonal antibody as $R_2$ - (with $R_3$ differing from $R_2$ only by virtue of being unlabeled).

The water-insoluble substrate that is used to capture $R_1$ may be prepared from inorganic and/or organic materials that will maintain their structural integrity when exposed to water or biological fluids. Suitable inorganic materials that may be used in preparing the water-insoluble substrate include siliceous materials {such as, glass (porous glass, frosted glass and so on), silica (silica gel, colloidal silica and so on), bentonite, wollastonite, cordierite, and the like}; and nonsiliceous metal oxides {such as alumina, spinel, apatite, hydroxy apatite, titania, zirconia and magnetic substances (such as iron oxides, ferrite, nickel oxides, cobalt oxides, and the like)}. Suitable organic materials that may be used in preparing the water-insoluble substrate include polymeric particles (such as, for example, polystyrene and derivatives thereof, acrylic polymers, polymethacrylates, polyolefins, halogen-containing polymers, polyesters, and polyamides), polysaccharides, cellulose, dextran, agarose, paper, polypeptide, collagen, and the like.

The water-insoluble substrates may be particulate in nature (varying from a finely divided powder, such as a magnetic ferrofluid, to a coarse granular material) or may be a shaped articles, such as beads, test tubes trays, microtiter plates, membranes, films, filter papers, discs, and so on.

Preferably, $R_1$ is immobilized on a water-insoluble particulate substrate having a particulate size ranging from 0.01 $\mu$m to 20 $\mu$m, more preferably from 0.1 $\mu$m to 1 $\mu$m, and most preferably from 0.2 $\mu$m to 1.2 $\mu$m.

Water-insoluble substrates particularly useful in this invention include particles prepared from polymers having reactive groups which will readily react with $R_1$. Examples of such reactive groups include active halogen groups, active ester groups, vinyl groups, aldehyde groups, aziridine groups, epoxy groups, carboxy groups, vinylsulfonyl, activated 2-substituted ethylsulfonyl, and primary amine groups. Useful methods of making polymers incorporating these reactive groups and methods for reacting these polymers with materials such as $R_1$ are well-known and described, for example, in EPA 0 323 692; EPA 0 496 472; EPA 0 496 472; EPA 0 466 220; EPA 0 308 235; EPA 0 426 670; and EPA 0 411 711. Also employable as insoluble substrates are core/shell polymers, as described in US-A-4,997,772, and polymer particles that can be dyed, as described in US-A-5,053,443 and in EPA 0 308 233, US-A-5,147,777; US-A-5,149,737; US-A-5,210,289; and EPA 91,201,419.8.

Among the water-insoluble substrates more preferred for the immobilization of $R_1$ are particles prepared from polymers containing activated 2-substituted ethylsulfonyl groups, particularly 2-halo ethylsulfonyl groups, or polymers containing reactive carboxy groups.

$R_1$ may be immobilized onto the water-insoluble substrate by any means known to those skilled in the art, including, for example, chemical coupling, simple adsorption, or employing specific binding pairs, as known in the art. For example, chemical coupling can involve treating the insoluble substrate with one or more chemical compounds (silanes, polyisocyanates and the like), followed by contacting the treated water-insoluble substrate with an aqueous solution of $R_1$. Alternatively, the substrate may have reactive groups for covalent coupling with $R_1$. As known in the art, for example, adsorption can be accomplished by contacting an aqueous solution of $R_1$ with the insoluble substrate for a time sufficient to permit the desired or maximum degree of immobilization. As described in EPA 0 302 715, specific binding pairs such as avidin and biotin, a lectin, and a sugar may also be employed.

Most preferably, when $R_1$ represents an antibody, the water-insoluble substrate is prepared from poly-[styrene-co-*m* and *p*-(2-chloroethylsulfonylmethyl)styrene] bead covalently bound to $R_1$ with techniques such as, for example, that described in EPA 0 323 692. Most preferably, when $R_1$ represents phosphorylcholine (preferably a modified phosphorylcholine having an amine or sulfhydryl group containing ester of phosphorylcholine, more preferably 4-aminophenyl ester of phosphorylcholine), the insoluble substrate is prepared from a carboxylic acid containing polymer, preferably a poly{styrene-co-3-(*p*-vinylbenzylthio)-propionic acid}, where $R_1$ is covalently bound to the polymer by a reaction of the carboxyl group of the polymer with either an amine of the modified phosphorylcholine or a sulfhydryl group of the modified phosphorylcholine (preferably the amine group) with the assistance of an activating agent, such as, for example, carbodiimides as described in US-A-4,421,847.

As illustrated by Comparative Example 2 (hereinafter), to reduce the saturation of the measurement signal system when the ligand is present in high concentrations a ratio of $R_2$:$R_3$ is used. $R_2$ and $R_3$ are present in the assay within the ratio of $R_2$:$R_3$ of 1:50 to 1:1000, preferably from 1:65 to 1:750, and most preferably from 1:80 to 1:155.

The assay may be carried out in any order, for example, $R_1$, $R_2$, and $R_3$ may be included such that the fluid sample interacts with all receptors simultaneously, or each receptor may be individually included separately, before, or after the introduction of the fluid sample.

$R_2$ is labeled with one or more suitable substances that are capable of generating a measurable signal either alone, or in conjunction with other reactants, as is known to those skilled in the art. Suitable substances that may be attached to $R_2$ as a label include, but are not limited to, one or more of the following: radioactive isotopes (such as $I^{125}$ and the like), enzymes (such as, beta-galactosidase, alkaline phosphates, glucose oxidase and horseradish peroxidase, and the like), fluorescent substances (such as, europium, europium derivatives, and the like), chemiluminescent substances (such as, for example, luminol derivatives), dyes (such as, for example, leuco dyes and fluorescent dyes), and so on. Preferred labels are enzymatic labels such as alkaline phosphates, glucose oxidase, and horseradish peroxidase (HRP). The most preferred label for $R_2$ is HRP.

Any number of techniques known to those skilled in the art may be employed in attaching the labeling substances to $R_2$. Preferably, the labeling substance is covalently bound to $R_2$ with any suitable technique.

When $R_2$ is labeled with an enzymatic substance, other reactants (typically a substrate for the enzyme) are included in the assay system. When the inventive assay is prepared as such, the substrate for the enzyme may be included in the assay system prior to the introduction of the liquid sample, simultaneously with the liquid sample or after completion of the binding reaction of the sandwich, as known to those skilled in the art. The enzymatic substrate may be a material which is directly acted upon by the enzyme label, or a material that is involved in a series of reactions which involve enzymatic reaction of the label. For example, if the enzyme label is a HRP, the substrate may be hydrogen peroxide. If the enzyme is glucose oxidase, the substrate is glucose.

Additionally, when enzymatic labels are used, an indicator composition may be employed in the assay system comprising one or more reagents which provide a detectable species as a result of reaction of the label. Preferably, the indicator composition is a colorimetric indicator which provides a colorimetrically detectable species as a result of enzymatic reaction with the substrate. The indicator composition may be a single compound or combination of reagents which produce a detectable dye or chemiluminescent emission of light upon the enzymatic reaction, as known in the art and described in US-A-4,729,950 and 4,598,044. For example, when glucose oxidase is used as the enzyme label, glucose may be used as the substrate to form gluconic acid upon a reaction, whereupon a colorimetric indicator composition may be employed that has a coupler and oxidizable compound which react with the gluconic acid to produce a dye. Alternatively, when HRP (or another suitable peroxidative compound) is used as the enzyme label, then a leuco dye which generates a detectable dye in the presence of peroxidase and hydrogen peroxide may be employed. Suitable leuco dyes include, but are not limited to those described in US-A-4,089,747; US-A-4,670,385; and US-A-4,828,983.

The method of this invention is adaptable both to solution assays and to dry analytical elements.

When the immunoassay is prepared as a solution assay, the analytical composition (and indicator composition if included) is contacted with a liquid sample containing the ligand in a suitable container (for example, test tube, petri dish, beaker, cuvette, and so on).

When the method of the invention is practiced with a dry analytical element, the simplest element can be composed of an absorbent carrier material such as, for example, a thin sheet of a self supporting absorbant or bibulous material, such as filter paper or strips, which contain the analytical composition. The element may also be divided into two or more discrete zones with different components of the analytical composition incorporated into individual zones of the materials. Such elements include test strips, diagnostic elements, dip sticks, diagnostic agents and the like, with procedures for their preparation and use known to those skilled in the art.

Dry analytical element formats especially useful in the practice of this invention are those having a porous spreading zone (prepared from porous particulate structures, porous polymeric films, porous reflective polymer beads, cellulose, glass fibers, woven and nonwoven fabrics, and the like) and a nonporous, transparent support (prepared from paper, metal foils, polystyrene, polyesters, polycarbonate, cellulose esters, gelatin, and so on).

It is desirable that the spreading layer be isotropically porous, having the same porosity in each direction of the zone. Such spreading zones are prepared from a number of materials including a structure of adhered particles as described, for example, in US-A-4,258,001 and pigmented zones such as those described in US-A-3,992,158. Interconnection of the particles comprising the bead spreading zone may be interconnected spaces or pores between particles, fibers, polymeric strands, and so on.

Preferred spreading zones are those described in US-A-3,992,158 as "blush polymer" zones. Such zones can be formed on a supporting material by dissolving a polymer in a mixture of two organic liquids,

one of which is a lower boiling, good solvent for the polymer and other being a high boiling, non-solvent or poor solvent for the polymer.

The resulting polymer solution is coated on the supporting material and dried under controlled conditions to leave an isotopically porous zone. Various polymers are known to be useful in this context including, but not limited to, polycarbonates, polyamides, polyurethanes and cellulose esters such as cellulose acetate (which is preferred).

Optionally within the porous zone may be incorporated particulate materials of various sizes to enhance the void volume. Useful particulate materials include, but are not limited to, inorganic pigments such as titanium dioxide, barium sulfate, zinc oxide, lead oxide with titanium dioxide being preferred. Further details of the preparation of "blush polymers" are described in US-A-3,992,158.

Preferably, the porous spreading zone is the outermost zone of the element (if more than one zone is used). The porous spreading zone may be prepared from any of the known materials used for such zones as described, for example in US-A-4,292,272, US-A-3,992,158, US-A-4,258,001 US-A-4,430,436 and related U.S. patents, and JP 57(1982)-101760. Preferably the particulate matter of the spreading zone is present in a coverage from 20 to 150 $g/m^2$, and more preferably from 50 to 150 $g/m^2$.

Preferably, the dry element of this invention has two or more contiguous zones (or layers) which are fluid permeable. The zones can be "self-supporting", which means that the zones can be composed of materials which maintain their integrity when exposed to aqueous fluids, such as filter papers or microporous membranes. Preferably, however, such zones are disposed on a separate, nonporous support which is dimensionally stable, inert to chemical reaction and preferably transparent (that is, radiation transmissive for wavelengths between 200 and 900 nm). However, non-transparent supports can be used if the mode of detection is reflectance spectroscopy instead of transmission spectroscopy. Useful supports are well known in the art, including but not limited to polyesters, papers, metal foils and polystyrene, polycarbonates and cellulose esters.

The preferred dry analytical elements may contain at least one other zone which contains one or more reagents needed for the assay. Such a zone is often known in the art as a reagent or registration zone, but in addition it can also include a second porous spreading zone if desired or printed layers located on other zones. The zones are generally in fluid contact with each other, meaning that fluids, reagents and reagent products can pass or be transported between superposed regions of adjacent zones, unless of course, a reagent is immobilized in some manner so it will not migrate within or without a zone, as known to those skilled in the art. Preferably, the zones are separately coated and superposed layers on an inert support (see Example 1 below). The reagent zones or layers can be composed of one or more binder materials (such as gelatin and other colloidal materials, hydrophilic polymers such as polyvinyl alcohol, polyacrylamide and others known in the art) in which reagents are incorporated.

The methods of preparing such elements are well known in the art and involve application of wet formulations of the zone composition onto a support and drying under suitable conditions. Coating procedures are well described in the art cited above for describing the spreading zones.

Although $R_1$, $R_2$ and $R_3$ may be placed in different or the same zone(s) within the element, a particularly preferred dry analytical element comprises a polymeric bead spreading zone wherein $R_1$ is incorporated into the zone. As incorporated, $R_1$ is immobilized on a water insoluble particulate substrate dispersed within the spreading zone, with said particulate substrate of the size (as previously described) falling with the range from 0.001 $\mu$m to 20 $\mu$m. Preferably $R_2$ and $R_3$ are incorporated in the element in a zone separate from the porous spreading zone, although $R_2$ and $R_3$ may both (or separately) be present in solution and not incorporated into the element (with contacting with element occurring before, during or after the fluid sample is contacted with the element).

It has been discovered that when CRP is the ligand to be measured, the performance of the assay is improved by including calcium ions to the assay system. For example calcium may be included in the fluid sample, in the solution assay, or incorporated into the dry analytical element. Preferably when included, the calcium ions are present within the range of from 0.01 M to 0.5 M of calcium, more preferably at a level from 0.02 M to 0.2 M and most preferably 0.1M. Any suitable form of calcium ions may be used, such as, calcium chloride, calcium acetate, calcium bromide, calcium iodide, calcium nitrate, and so on, (preferably in solution), as widely commercially available. A convenient form, because of cost and availability, of the calcium ions is $CaCl_2 \bullet H_2O$.

It has also been found that when CRP is the ligand to be measured the measurement of the assay system is enhanced by using SDS as a solvent when employing triarylimidazole leuco dye as a reagent. More preferably, when a dry element format is employed, the SDS and leuco dry solution is incorporated into the element as an emulsion (preferably into a reagent zone separate from the spreading zone, more preferably into a gelatin reagent zone) in a manner, for example, as shown in Example 3 below.

As known to those skilled in the art, the specimen fluid sample may be physically contacted with the assay system by many methods, for example using an amount of fluid sample from 1 to 200 µl. After contact, the sample and reagents within the element become mixed within the various zones when more than 1 zone is employed. Such contact can be accomplished in any suitable manner, for example by dipping or immersing the element into the sample, or preferably, by spotting the sample onto the element by hand or suitable machine.

To obtain the water-insoluble complex of $R_1$-ligand-$R_2$ (hereinafter "water-insoluble complex") after the specimen is introduced to the assay system, the assay may be carried out under usual conditions, as known to those skilled in the art. For example, after sample application, the assay may be exposed to any conditioning, such as incubation, heating or otherwise, that may be desirable to quicken or otherwise facilitate obtaining a test result. Preferably, the assay may be accomplished within a temperature range of from 5-50°C (preferably 34-40°C) for a period of a few seconds to 24 hours. Although the assay may be incubated over a wide pH range, typically the assay may be carried out within the pH range from 5 to 9.

The concentration of the ligand may be measured by quantifying the portion of $R_2$ present in the water insoluble complex or the unreacted $R_2$ which is left unbound and soluble.

Physical separation of the water insoluble complex from the fluid sample and the unreacted $R_2$ may be accomplished with any suitable technique as known to those skilled in the art. For example, after the reaction period, the excess unbound $R_2$ may be removed from the water insoluble complex by a washing step by any suitable technique, as known to those skilled in the art.

When utilizing the washing step, the wash fluid employed may be any suitable solution, as known to those skilled in the art. For example, a preferred wash fluid includes distilled water, physiologic saline, phosphate buffer (such as for example $Na_2HPO_4$ or $NaH_2PO_4$), and the like. More preferably, when the label is an enzyme, the wash solution may also include, for example, a substrate specific for the enzyme, a surfactant [preferably a nonionic surfactant such as, for example, poly(oxyalkylene alcohols)], an electron transfer agent to assist in the oxidation of substrate (including, for example, p,p'-biphenol, p-methoxyphenol, o-methoxyphenol, p-anisidine, p-hydroxy-N,N'-dimethylaniline, or o-phenylenediamine, and 4'-hydroxyacetanilide), and a complexing agent [such as, for example, acid derivatives including acetic acid derivatives of di-, tri-, and tetra-amino compounds (such as ethylenediamine tetraacetic acid and diethylenetriamine pentaacetic acid)]. Particularly effective as a wash fluid when the label is a horseradish peroxidase and the colorimetric indicator is a leuco dye, is a phosphate buffered solution of hydrogen peroxide (the substrate) with an electron transfer agent (most preferably 4'-hydroxyacetanilide) to assist in oxidation of the hydrogen peroxide, and a complexing agent (most preferably diethylenetriamine pentaacetic acid). Other assay formats may also be used, such as, for example, those described in EPA 0 462 644.

Detection of $R_2$ for purposes of measuring the concentration of the ligand may be accomplished by methods known to those skilled in the art, including, for example, measuring the signal of the immunoassay through radioimmunoassay, enzyme immunoassay, fluorescence immunoassay, chemiluminescence immunoassay, and the like. Although the inventive assay is especially well-suited for quantitative analysis, the assay is equally well suited for qualitative analysis.

The present invention is now further illustrated by, but is by no means limited to, the following examples.

**EXAMPLES**

In each of the examples, the assays employed a dry analytical element and wash solution. The leuco dye was the triarylimidazole leuco blue color dye 4,5-Bis (4-dimethylaminophenyl)-2-(4-hydroxy-3,5-dimethoxyphenyl)imidazole. As noted in the elements, q.s. indicates the amount of deionized, distilled water needed to fulfill the total wet coverage for each layer described. Other materials used for the element and wash solution were obtained as follows: SURFACTANT 10G (a isononylphenoxypolyglycidol surfactant) supplied by Olin Corporation (Stamford, Conn., U.S.A.), TRITON™ X-100 nonionic surfactant supplied by Rohm and Haas (Philadelphia, Pa., U.S.A.), ZONYL™ FSN nonionic surfactant supplied by DuPont (Wilmington, Del., U.S.A.), horseradish peroxidase supplied by Sigma Chemical Co. (St. Louis, Mo., U.S.A) or Miles Laboratories (Elkhart, Ind., U.S.A.), and the remainder of the components described below in the film element or wash solution supplied by either Eastman Kodak Company (Rochester, N.Y., U.S.A.) or are preparable using standard procedures and readily available starting materials, as known to those skilled in the art.

ELEMENT A

| LAYER | COMPONENT | COVERAGE (g/m$^2$) |
| --- | --- | --- |
| | TOTAL WET COVERAGE | 270 |
| | Deionized distilled water | q.s. |
| -02 BEAD SPREADING LAYER | N-[Tris(hydroxymethyl)methyl]-2-aminoethanesulfonic acid buffer (pH 7.55) | 0.219 |
| | 5,5-Dimethyl-1,3-cyclohexanedione | 0.075 |
| | 4,5-Bis(4-dimethylaminophenyl)-2-(4-hydroxy-3,5-dimethoxyphenyl)imidazol leuco dye | 0.300 |
| | Dimethyl sulfoxide | 2.700 |
| | Poly (methyl acrylate-co-sodium 2-acrylamido-2-methylpropanesulfonate-co-2-acetoacetoxyethyl methacylate) | 2.583 |
| | Poly(*m*- and *p*-vinyltoluene-co-methacrylic acid) particles having a an average diameter of 30 µm | 130.0 |
| | ZONYL FSN | 0.054 |
| | Methanol | 0.675 |
| | Particles of poly[styrene-co-*m*-& *p*-(2-chloroethylsulfonylmethyl) styrene] covalently bound to CRP polyclonal antibody | 1.2 |
| | Isopropyl alcohol | 0.405 |
| | SURFACTANT 10G | 0.238 |
| -01 GEL | TOTAL WET COVERAGE | 100 |
| | Distilled deionized water | q.s. |
| | Bone gelatin | 10.00 |
| | 4'-hydroxyacetanilide | 0.15 |
| | N-[Tris(hydroxymethyl)methyl-2-aminoethanesulfonic acid buffer | 4.58 |
| | TRITON X-100 | 0.020 |

| ELEMENT A -Continued- | | |
|---|---|---|
| | Bis(vinylsulfonylmethyl) ether | 0.15 |
| SUPPORT | Poly(ethylene terephthalate) | |

WASH FLUID FORMULA

| | |
|---|---|
| Hydrogen Peroxide | 0.03 % |
| Sodium Phosphate, pH 6.8 | 0.01 M |
| 4'-Hydroxyacetanilide | 0.005 M |
| Diethylenetriaminepentaacetic acid | 10 µM |
| Distilled deionized water | q.s. |

The first layer of the element (layer 01) was coated on subbed, gel-washed poly(ethylene terephthalate) support, with the second layer (layer 02) coated on top.

$R_1$ was a polyclonal anti-CRP goat antibody (purchased from DiaMed Company, South Windham, ME, U.S.A.) that was covalently bonded to particulate water insoluble polymeric beads of poly[styrene-co-*m* &-*p*-(2-chloroethylsulfonylmethyl)styrene], as described in EPA 0 302 715 B1 and US-A-5,177,023, with a weight ratio of 90/10; size 0.42 µm diameter.

Example 1

Fluid samples of the antigen CRP (purchased from Scripps Laboratories) were prepared in concentrations varying from no CRP (the control) to 25.6 mg/l of CRP (as shown in TABLE I hereinafter) in a solution of sodium phosphate (0.02 M having a pH of 6.8) and sodium chloride (0.15 N). $R_2$ was a monoclonal anti-CRP antibody (purchased from Medix Biochemica, Finland) having a horseradish perioxidase label attached thereto by a thiol/maleimide procedure, as described in Clinical Chemistry, vol. 30, pp. 1446-1451 (1984). $R_3$ was an unlabeled monoclonal anti-CRP antibody (identical to $R_2$ but having no HRP label). $R_2$ and $R_3$were included in the CRP solutions in a ratio of $R_2$:$R_3$ of 1:151 as follows: $R_2$ = 4.455 x $10^{-3}$ µg (6.46 x $10^{-4}$ IU, 1 µg = 0.145 IU, IU= International Units of HRP activity) and $R_3$ = 0.675 µg. A total of 10 µl of the CRP, $R_2$, and $R_3$ solution was spotted on a film element for each concentration of CRP. The spotted elements were then incubated at 37°C for 5 minutes and washed thereafter with 10 µl of the wash solution described above to move the excess labeled $R_2$ to the edges of the element and initiate the HRP-catalyzed dye oxidation.

The elements were then placed back in the incubator at 37°C. The amount of immuno complex of $R_1$-CRP-$R_2$ sandwich was determined by measuring the rate of the triarylimidazole leuco dye oxidation by hydrogen peroxide in the center of the element in about a 0 to 1 minute read time window.

The rate of increase of the transmission density ($D_t$/min) of each washed element was measured by a reflectometer as described in J. Optical Soc. Am., 43, 595 (1953). Data are recorded in TABLE I below with calibration curve of the data shown in the *Fig.* as line 1. As shown, a measurement over the entire dynamic range was possible without saturation of the measurement signal.

Comparative Example 1

Fluid samples of CRP were prepared in the concentrations as described in Example 1. This example demonstrates a comparison, where only $R_2$ is employed in the immunoassay at a concentration of 4.455 x $10^{-3}$ µg [6.46 x $10^{-4}$ IU]. $R_3$ was not included.

A total of 10 µl of the CRP and $R_2$ solution was spotted on the film element for each concentration of CRP. The spotted elements were then incubated, washed and measured as described in Example 1. The data are recorded in TABLE I, hereinafter, with a calibration curve of the data shown in the *Fig*. as line 2. As the calibration curve indicates, the read out quickly becomes saturated, thus indicating an inadequate dynamic range for CRP.

Comparative Example 2

Fluid samples of CRP were prepared in the concentrations as described in Example 1. This example demonstrates a comparison, where the $R_2$ and $R_3$ are employed in a ratio of 1:0.291 (3.43:1), as follows: $R_2$ = 4.455 x $10^{-3}$ µg (6.46 x $10^{-4}$ IU) and $R_3$ = 1.299 x $10^{-3}$ µg, as taught by Tung, et al. in US-A-4,788,138.

A total of 10 µl of the CRP, $R_2$, and $R_3$ solution was spotted on the film element for each concentration of CRP. The spotted elements were then incubated, washed and measured as described in Example 1. The data are recorded in TABLE I, hereinafter, with a calibration curve of the data shown in the *Fig*. as line 3. As the calibration curve indicates, the read out quickly became saturated although $R_3$ (excess antibody) was employed, thus indicating that the dynamic range is not improved by the excess antibody for purposes of measuring CRP.

**TABLE I**

**(Example 1 and Comparative Examples 1 & 2)**

Lines (as corresponding to *Fig.*)

1. $R_2 + R_3$ (Ratio of $R_2$:$R_3$ = 1:151), [$R_2$ = 4.455 x $10^{-3}$ µg (6.46 x $10^{-4}$ IU), $R_3$ = 0.675 µg] --Example 1
2. $R_2$ only --Comparative Example 1
3. $R_2 + R_3$ [Ratio of $R_2$:$R_3$ = 1:0.291 (3.45:1)] [$R_2$ = 4.455 x $10^{-3}$ µg (6.46 x $10^{-4}$ IU)] --Comparative Example 2

| Lines | 1 | 2 | 3 |
|---|---|---|---|
| CRP (mg/l) | Signal ($D_t$/minute) | | |
| 0 | 0.00656 | 0.01543 | 0.01136 |
| 0.35 | 0.01019 | 0.17207 | 0.09573 |
| 2.03 | 0.02021 | 0.18413 | 0.16234 |
| 4.6 | 0.03814 | 0.16039 | 0.14849 |
| 9.2 | 0.06085 | 0.14558 | 0.13583 |
| 12.8 | 0.06295 | 0.13850 | 0.13464 |
| 18.4 | 0.08302 | 0.11367 | 0.10235 |
| 25.6 | 0.12621 | 0.01521 | 0.08963 |

The data shown in TABLE I illustrate the measurement of an antigen over a wide range concentration. The unexpected advantage of employing $R_2$:$R_3$ within the ratio taught by this invention is shown in the *Fig.* Only Example 1 (Line 1 in the *Fig.*) demonstrates a measurement of the CRP over the entire range of concentration tested. Example 2 (Line 2) is included to show a typical immunoassay of the prior art having only $R_2$, where the read out quickly becomes saturated. Example 3 (Line 3 in the *Fig.*) illustrates an adjusted immunoassay having an excess unlabeled antibody ($R_3$) outside the ratio taught by the present invention. Unexpectedly, the ratio of $R_2$:$R_3$ has been found to be important in improving the dynamic range of specific binding assays.

Example 2

Elements were prepared in accordance with the procedures described in Example 1, with the exception that 0.0045 CRP monoclonal antibody ($R_2$) and 0.000053 CRP monoclonal antibody ($R_3$) were incorporated into the bead spreading layer of the film element rather than being included in the CRP solutions at a ratio of $R_2$:$R_3$ of 1:86. The concentration of CRP in the fluid samples tested as well as the signal measurement for each sample tested is shown in TABLE II. As the data in TABLE II indicate, saturation of the measurement signal did not occur at high concentrations of the antigen CRP.

TABLE II

| (Example 2) | |
|---|---|
| CRP (mg/l) | Signal Dt/minute |
| 0 | 0.028 |
| 0.68 | 0.050 |
| 5.4 | 0.147 |
| 15.0 | 0.223 |

Example 3

The procedures of Example 2 were followed using ELEMENT B shown hereinafter.

ELEMENT B

| LAYER | COMPONENT | COVERAGE ($g/m^2$) |
| --- | --- | --- |
| | TOTAL WET COVERAGE | 270 |
| | Deionized distilled water | |
| -02 BEAD SPREADING LAYER | N-[Tris(hydroxymethyl)methyl]-2-aminoethanesulfonic acid buffer pH = 7.0 | 0.219 |
| | ZONYL FSN | 0.054 |
| | Methanol | 0.675 |
| | 5,5-Dimethyl-1,3-cyclohexanedione | 0.5 |
| | Bovine serum albumin | 0.5 |
| | $CaCl \cdot H_2O$ | 1.48 |
| | 4'-hydroxyacetanilide | 0.15 |
| | Poly[styrene-co-3-(p-vinylbenzyl-thio)propionic acid] having phosphorylcholine covalently bound thereto ($R_1$) | 0.16 |
| | Poly(*m*- and *p*-vinyltoluene-co-methacrylic acid) particles having an average diameter of 30 µM | 130.0 |
| | Monoclonal antibody ($R_3$) | 0.027 |
| | Monoclonal antibody covalently bound to horseradish peroxidase ($R_2$) | 0.00035 |
| -01 GEL | TOTAL WET COVERAGE | 139 |
| | Distilled deionized water | 139 |
| | Bone gelatin | 5 |
| | N-[Tris(hydroxymethyl)methyl-2-aminoethanesulfonic acid buffer pH = 7.0 | 4.58 |
| | 4'-Hydroxyacetanilide | 0.15 |
| | 4,5-Bis(4-dimethylaminophenyl)-2-(4-hydroxy-3,5-dimethoxyphenyl)imidazol | 0.15 |
| | Leuco dye and sodium dodecyl sulfate emulsion | 1.34 |

| Element B --Continued-- | | |
|---|---|---|
| • | Bis(vinylsulfonylmethyl) ether | 0.15 |
| | TRITON X-100 | 0.004 |
| SUPPORT | Poly(ethylene terephthalate) | |

This example demonstrates both an immuno reaction and a non-immuno mechanism where $R_1$ is a 4-aminophenyl ester of phosphorylcholine of the following structure:

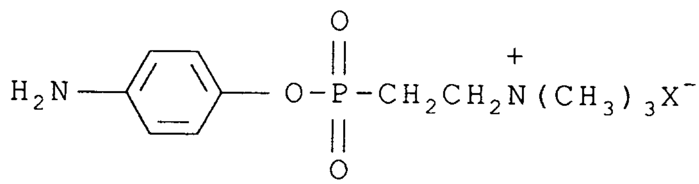

that is attached to the particulate insoluble substrate of poly[styrene-co-3-(*p*-vinylbenzylthio)propionic acid] beads. The beads (0.4 μM diameter) were covalently bound to $R_1$ by reacting the amine group with the carboxy reactive groups of the polymer backbone using a carbodiimide activating agent [1-(3-dimethylaminopropyl)-3-ethylcarbodiimide,as described in US-A-4,421,847] for about 4 hours. $R_2$ and $R_3$ were incorporated into the element at a ratio of 1:77 ($R_2$:$R_3$). Calcium was incorporated into the bead spreading layer at 1.48 g/m$^2$. The procedures of Example 1 were followed in measuring varying concentrations of CRP fluid samples, with the concentration levels as well as the measurement of the signal generated shown in TABLE III hereinafter.

Table III

| (Example 3) | |
|---|---|
| CRP (mg/l) | Signal Dt/minute |
| 1.5 | 0.1264 |
| 4.3 | 0.1348 |
| 8.2 | 0.1523 |
| 17.4 | 0.2034 |
| 36.1 | 0.2923 |

## Claims

1. A specific binding assay method comprising: (a) contacting a liquid sample containing a ligand to be measured with (i) a first receptor $R_1$ specific for the ligand, $R_1$ being immobilized on a water-insoluble substrate; (ii) a labeled, unbound second receptor $R_2$ specific for the ligand; and (iii) an unlabeled, unbound third receptor $R_3$ specific for the ligand, to obtain a water-insoluble complex of $R_1$, ligand, and $R_2$; (b) separating the water-insoluble complex from the liquid sample and unreacted $R_2$; and (c) measuring either the amount of $R_2$ associated with the water-insoluble complex or the amount of unreacted $R_2$ as an indication of the amount of the ligand in the sample,
   characterised in that $R_2$ and $R_3$ are present in a ratio of $R_2$:$R_3$ of from 1:50 to 1:1000 and $R_3$ is the same as $R_1$, $R_2$, or a mixture thereof.

2. A method according to claim 1 characterised in that the ligand to be measured is C-Reactive Protein.

**3.** A method according to either claim 1 or claim 2 characterised in that $R_1$ is phosphorylcholine, $R_2$ is an antibody specific to the ligand, and the insoluble support is a carboxy containing polymeric particle.

**4.** A method according to any one of claims 1, 2, or 3 characterised in that $R_3$ is unbound phosphorylcholine.

**5.** A method according to any one of claims 1, 2, or 4 characterised in that $R_3$ is unlabeled $R_2$.

**6.** A method according to any one of claims 1 to 5 characterised in that it further comprises attaching the water-insoluble substrate $R_1$ by using a carbodiimide as an activating agent.

**7.** A method according to any one of claims 1 to 6 characterised in that the label on $R_2$ is an enzyme.

**8.** A method according to claim 7 characterised in that the enzyme label is horseradish peroxidase.

**9.** A method according to any one of claims 1 to 8 characterised in that the ratio of $R_2$:$R_3$ is within the range of 1:80 to 1:155.

**10.** An analytical element comprising a porous spreading zone and in the porous spreading zone, or a different zone, (a) a first receptor $R_1$ specific to the ligand, $R_1$ being immobilized on a water-insoluble substrate; (b) a labeled, unbound second receptor $R_2$ specific for the ligand; and (c) an unlabeled, unbound third receptor $R_3$ specific for the ligand,
characterised in that $R_2$ and $R_3$ are present in a ratio of $R_2$:$R_3$ of from 1:50 to 1:1000, and $R_3$ is the same as $R_1$, $R_2$, or a mixture thereof.

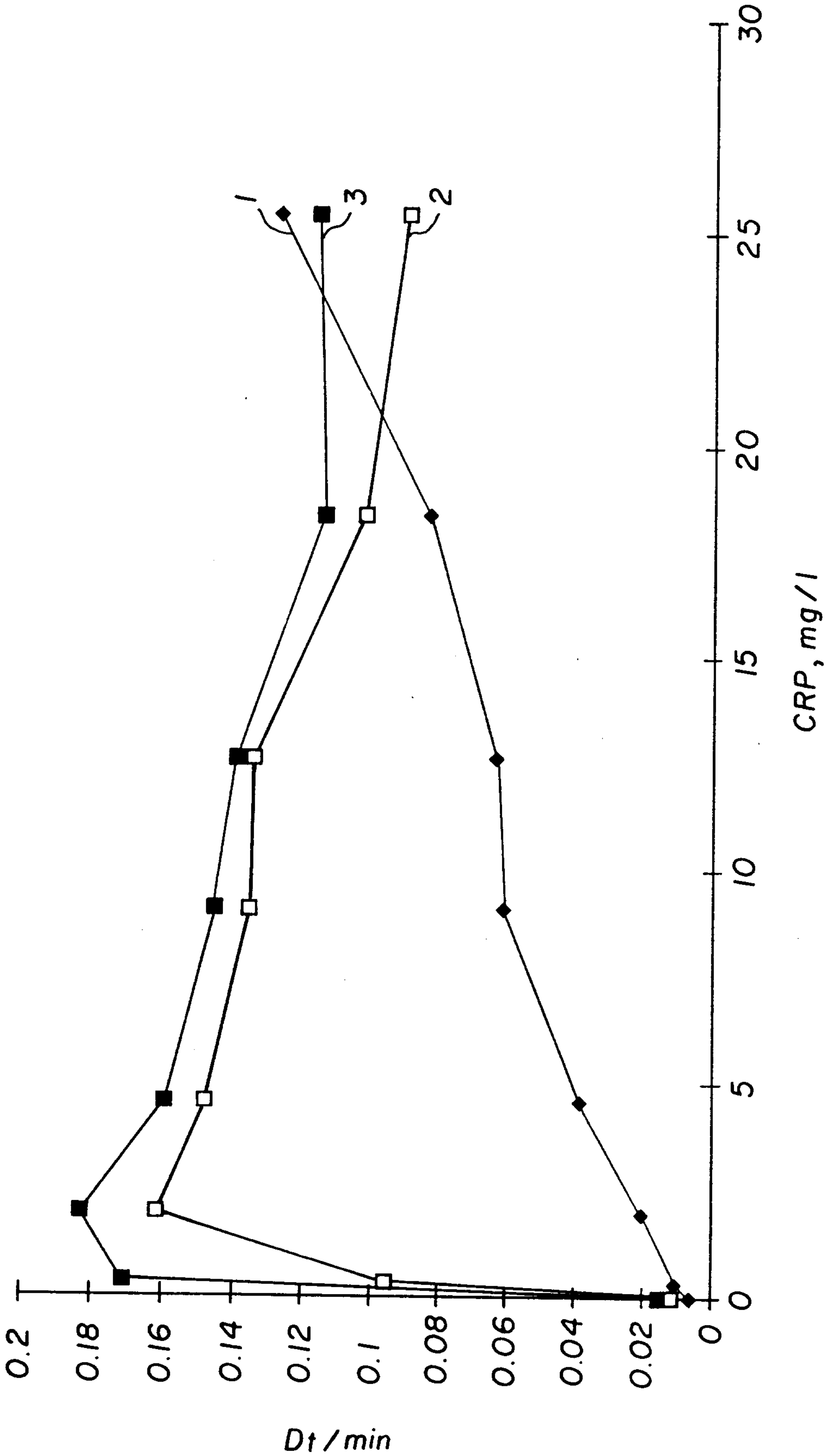

Dt / min
0.2
0.18
0.16
0.14
0.12
0.1
0.08
0.06
0.04
0.02
0
CRP, mg/l
0
5
10
15
20
25
30
1
2
3

European Patent Office

# EUROPEAN SEARCH REPORT

Application Number

EP 93203559.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | US - A - 4 743 542 (GRAHAM, JR. et al.) * Totality * | 1-10 | G 01 N 33/53<br>G 01 N 33/546<br>G 01 N 33/543 |
| D,A | US - A - 4 595 661 (CRAGLE et al.) * Abstract; claims * | 1-10 | |
| | | | TECHNICAL FIELDS SEARCHED (Int. Cl.5) |
| | | | G 01 N 33/00 |

The present search report has been drawn up for all claims

| Place of search | Date of completion of the search | Examiner |
|---|---|---|
| VIENNA | 24-03-1994 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)